# EUROPEAN PATENT APPLICATION

(11) **EP 2 014 646 A2**
(43) Date of publication of application: **14.01.2009**
(21) Application number: 08158056.5
(22) Date of filing: 11.06.2008
(51) Int. Cl.: C07C 381/00

(54) **Oxopentafluorosulfanyl-substituted alicyclic compounds**

(30) Priority: 12.06.2007 US 761447
(71) Applicant: Air Products and Chemicals, Inc., Allentown, PA 18195-1501 (US)
(72) Inventor: Syvret, Robert, George, Allentown, PA 18103-5451 (US); Lal, Gauri, Sankar, Whitehall, PA 18052 (US)
(74) Representative: Muir, Benjamin M. J.

(57) **Abstract**

A compound comprising at least one alicyclic or heteroalicyclic ring structure, the ring structure comprising a ring of about four to about eight atoms, wherein at least two adjacent atoms of the ring are carbon atoms and at least one of said carbon atoms is covalently bonded to an -OSF₅ functional group and either is bonded to a moiety selected from hydrogen; halogen; C₁ - C₆ substituted or unsubstituted, branched or straight alkyl; C₅ - C₇ substituted or unsubstituted aryl or heteroaryl; C₃ - C₈ aliphatic cyclic; OSF₅; or is a member of a fused, bridged, fused-bridged, or spirocyclic ring system; provided that if the moiety is fluorine, then at least one of the other ring atoms are bonded to an atom or functional group other than fluorine; as well as methods for making the same.

## Description

### BACKGROUND OF THE INVENTION

This invention relates to novel, oxopentafluorosulfanyl-substituted compounds and methods of producing the same.

Compounds having oxopentafluorosulfanyl (-OSF₅) functionality are useful in a wide range of applications such as electronics, pharmaceuticals, and polymers. Adding or substituting an oxopentafluorosulfanyl functional group on a compound can, in certain cases, advantageously modify certain properties of the compound. For example, the presence of this functional group can potentially have an effect on the compound's electronegativity, lipophilicity, oxidation resistance, thermal stability, chemical stability, steric bulk, selectivity in chemical transformations, dielectric properties, and/or biological activity. In certain applications, an oxopentafluorosulfanyl substituent is an environmentally friendly alternative to certain other substitutes, such as trifluoromethyl, because the oxopentafluorosulfanyl-based compound is more biodegradable. Substituting an oxopentafluorosulfanyl group for a trifluoromethyl on a biologically active compound can also improve the compound's efficacy, and in some instances, achieve new biological activity. In addition, a polymer's chemical resistance, stain resistance, etc., can be improved by incorporating oxopentafluorosulfanyl into the polymer chain.

Synthesis of oxopentafluorosulfanyl-substituted alkyl, aryl, and perfluorinated alicyclic compounds is known in the art. For example, pentafluorosulfur hypochlorite (SF₅OCl) has been shown to react with unsubstituted olefins, such as ethylene, or perfluorinated olefins, such as perfluoroethylene, to produce 1-oxopentafluorosulfanyl-2-fluoroethane and 1-oxopentafluorosulfanyl-1,1,2,2,2-pentafluoroethane, respectively. L. R. Anderson, et al., PERHALOALKYL HYPOCHLORITES AND PENTAFLUOROSULFUR HYPOCHLORITE - REACTIONS WITH OLEFINS, J. Org. Chem, Vol. 35, No. 11, 1970, p. 3730 - 73. DE 100 58 472 (Kirsch, et al.) discloses that reacting bis-pentafluorosulfur peroxide (SF₅OOSF₅) with bromobenzene produces 4-bromo-1-oxopentafluorosulfanylbenzene. Also, pentafluorosulfur hypofluorite (SF₅OF) has been shown to react with perfluorocyclopentene (C₅F₈) to form oxopentafluorosulfanyl-nonafluorocyclopentane. S. Williamson, et al., REACTIONS OF PENTAFLUOROSULFUR HYPOFLUORITE, Inorganic Chemistry, Vol. 1, No. 3, Aug. 1962, p. 673 - 77.

Notwithstanding these oxopentafluorosulfanyl-substituted alkyls, aryls, and perfluoronated alicyclics, applicants have come to appreciate the need for, and advantage of, oxopentafluorosulfanyl-substituted compounds which heretofore have not been known.

### BRIEF SUMMARY OF THE INVENTION

Applicants have found novel compounds having an alicyclic and/or heteroalicyclic ring with oxopentafluorosulfanyl functionality. Preferred compounds according to the invention are surprisingly stable when exposed to ambient conditions, such as ambient temperature and pressure, light, air, and moisture. As used herein, the term "stable" with respect to a compound means that the compound is not subject to sudden oxidation or decomposition under the described conditions.

Applicants have also found methods of producing compounds having an alicyclic and/or heteroalicyclic ring with oxopentafluorosulfanyl functionality. In preferred embodiments, the methods comprise reacting at least one compound having a cycloalkene or heterocycloalkene ring structure with a pentafluorosulfur hypohalite to form a compound having an oxopentafluorosulfanyl-substituted alicyclic or heteroalicyclic ring, respectively. Preferred methods involve a reaction that is not subject to sudden or extreme oxidation or decomposition. The stability of these reactions is surprising in view of the highly reactive nature of the reactants, particularly cycloalkenes and hypofluorites.

One aspect of the invention is a novel compound comprising at least one alicyclic or heteroalicyclic ring structure, the ring structure comprising a ring of four to eight atoms, wherein at least two adjacent atoms of the ring are carbon atoms and at least one of said carbon atoms is covalently bonded to a hydrogen atom and to an - OSX₅ functional group, where each X is independently F or Cl.

Another aspect of the invention provides a method for preparing a compound having oxopentafluorosulfanyl functionality comprising (a) providing a compound having a substituted or unsubstituted cycloalkene or heterocycloalkene ring; and (b) reacting said compound with a pentafluorosulfur hypohalite to form a compound having an oxopentafluorosulfanyl-substituted alicyclic or heteroalicyclic ring.

In certain embodiments, the present methods for adding an oxopentafluorosulfanyl group to a compound comprise (a) contacting a nonaqueous solution comprising a substituted or unsubstituted cycloalkene or heterocycloalkene with a pentafluorosulfur hypochlorite vapor, preferably at a temperature from -90° C to 0° C, to form a reaction mixture; and (b) maintaining said reaction mixture under conditions effective to form an oxopentafluorosulfanyl-substituted alicyclic or heteroalicyclic compound. In certain preferred embodiments, these conditions involve maintaining the reaction mixture at a temperature of from -90° C to 30° C for a time sufficient to form the oxopentafluorosulfanyl-substituted alicyclic or heteroalicyclic compound

According to a third aspect, the present invention provides a compound comprising at least one alicyclic or heteroalicyclic ring structure, the ring structure comprising a ring of four to eight atoms, wherein at least two adjacent atoms of the ring are carbon atoms and at least one of said carbon atoms is covalently bonded to an - OSF₅ functional group and either is bonded to an additional moiety selected from hydrogen; halogen; C₁ - C₆ substituted or unsubstituted, branched or straight alkyl; C₅ - C₇ substituted or unsubstituted aryl or heteroaryl; C₃ - C₈ aliphatic cyclic; OSF₅; or is a member of a fused, bridged, fused-bridged, or spirocyclic ring system; provided that if the additional moiety is fluorine, then at least one other ring atom is bonded to an atom or functional group other than fluorine.

According to a fourth aspect, the present invention provides a compound having a structure according to Formula I: 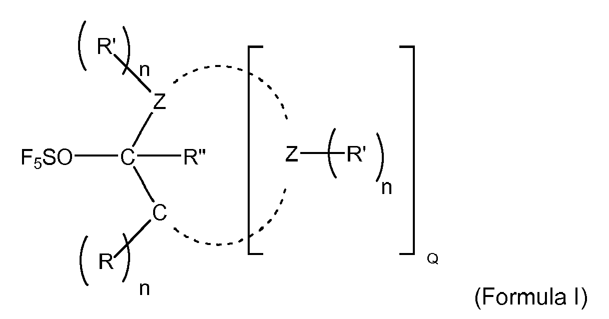 wherein
Z is independently selected from C, O, S, or N;
R, R', and R" are independently selected from H; halogen; C₁ - C₆ substituted or unsubstituted, branched or straight alkyl; C₅ - C₇ substituted or unsubstituted aryl or heteroaryl; C₃ - C₈ aliphatic cyclic; atom of a fused, bridged, fused-bridged, or spirocyclic ring structure; O; and OSF₅; provided that R" is not O, that if R' is O or OSF₅, the corresponding Z is C, and further that R, R', and R" are not all F;
Q is a positive integer from 1 to 5;
n is independently 0, 1, or 2; and
---- is independently a single or double covalent bond.

In one embodiment, at least one of said R' is halogen.

In one embodiment, R, R', and R" are independently selected from H and halogen. Preferably, said halogen is independently selected from F, Cl, and Br.

In one embodiment, at least one pair of two adjacent Z's in said ring structure are covalently bonded via a double bond.

In a preferred embodiment, the ring structure is of a monocyclic system. Preferably, the ring structure is a five- or six-member alicyclic or heteroalicyclic. More preferably, the ring structure is a six-member alicyclic. In a particularly preferred embodiment, the compound has a structure according to Formula II: 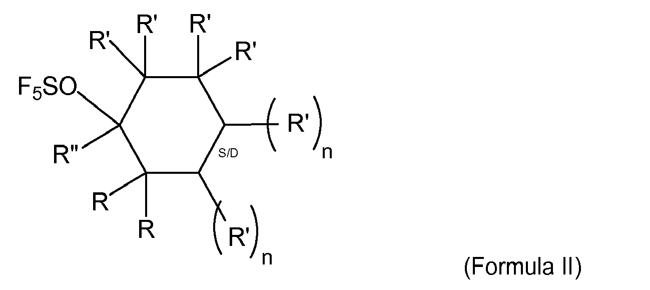 wherein
R, R', and R" are independently selected from H, F, Cl, Br, I, and OSF₅, and more preferably are independently selected from H, F, Cl, and Br; provided that at least one R, R', or R" is not F;
n is 1 or 2; and
^{S/D} is a single or double bond.

Exemplary compounds include:
3,4-dibromo-1-oxopentafluorosulfanyl-cyclohexane;
2-chloro-4,5-dibromo-1-oxopentafluorosulfanylcyclohexane;
2,4,5-trichloro-1-oxopentafluorosulfanylcyclohexane;
1-oxopentafluorosulfanyl-cyclohex-3-ene; and
2-chloro-1-oxopentafluorosulfanyl-cyclohex-4-ene.

In preferred embodiments, the compound is stable at ambient temperature, is stable when subjected to moisture, and/or is stable when subjected to air.

According to a fifth aspect, a method for preparing an oxopentafluorosulfanyl substituted compound is provided comprising:
a. providing a reactant comprising a compound having a substituted or unsubstituted cycloalkene or heterocycloalkene ring, provided that said ring is not perfluorinated;
b. reacting said reactant with a pentafluorosulfur hypohalite to form a compound having an oxopentafluorosulfanyl-substituted alicyclic or heteroalicyclic ring.

In a preferred embodiment said reacting involves an electrophilic addition across a pair of double bonded carbons in said cycloalkene.

The pentafluorosulfur hypohalite preferably has the formula of X'OSF₅, wherein X' is F or Cl. More preferably, the pentafluorosulfur hypohalite is pentafluorosulfur hypochlorite.

The reactant preferably comprises a compound having a four- to eight-member ring structure wherein said members are independently selected from C, N, O, and S. In one embodiment, the reactants comprises a substituted or unsubstituted cyclohexene or a substituted or unsubstituted cyclohexadiene.

The alicyclic or heteroalicyclic ring preferably has a structure according to Formula V: 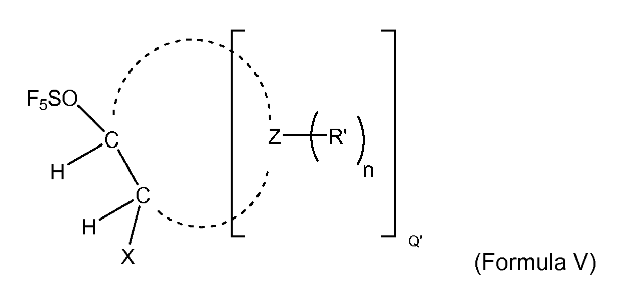 wherein
Z is independently selected from C, O, S, or N;
X is a halogen;
R' is independently selected from H, halogen, C₁ - C₆ substituted or unsubstituted, branched or straight alkyl, C₅ - C₇ substituted or unsubstituted aryl or heteroaryl, C₃ - C₈ aliphatic cyclic, atom of a fused, bridged, fused-bridged, or spirocyclic ring structure, O, and OSF₅, provided that if R' is O or OSF₅, the corresponding Z is C;
Q' is a positive integer from 2 to 6;
n is independently 1 or 2; and
---- is independently a single or double covalent bond.

In a preferred embodiment of the method, the reactant comprises a cycloalkene having a structure according to Formula IV: 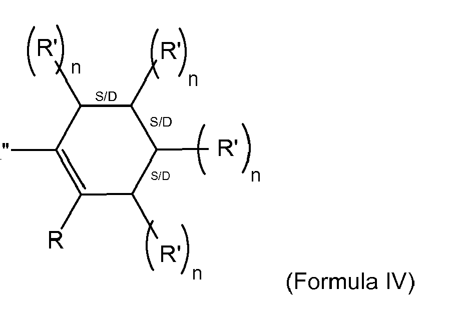 the pentafluorosulfur hypohalite is F₅SOCl, and
the alicyclic or heteroalicyclic ring has a structure according to Formula VI: 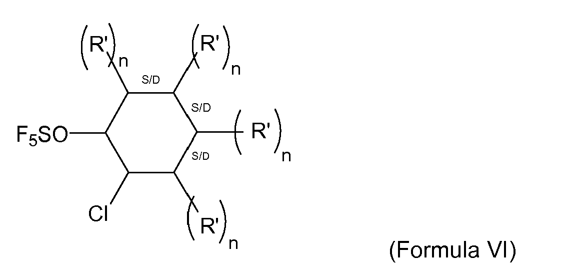 wherein
R and R" are hydrogen,
R' is independently selected from H, halogen, C₁ - C₆ substituted or unsubstituted, branched or straight alkyl, C₅ - C₇ substituted or unsubstituted aryl or heteroaryl, C₃ - C₈ aliphatic cyclic, and OSF₅;
n is independently 1 or 2; and
S/D is independently a single or double bond, provided that the ring does not contain an aromatic bond.

In said above embodiment, the method may further comprise: a dehalogenating process wherein at least one halogen is removed from a ring member; a dehalogenating process wherein a double covalent bond is formed between two adjacent ring members; and/or a halogenation process wherein at least one halogen is added to one or more ring members.

According to a yet further aspect, the present invention provides a method for adding an oxopentafluorosulfanyl group to a compound comprising:
a. contacting a nonaqueous solution comprising a substituted or unsubstituted cycloalkene with a pentafluorosulfur hypochloride vapor at a temperature from -90° C to 0° C to form a reaction mixture; and
b. maintaining said reaction mixture at a temperature of from -90° C to 30° C to form an oxopentafluorosulfanyl-substituted alicyclic compound.

### DETAILED DESCRIPTION OF THE INVENTION

One aspect of the invention provides novel organic compounds having at least one oxopentafluorosulfanyl-substituted alicyclic or heteroalicyclic ring structure.

The oxopentafluorosulfanyl substituent of the novel compound is bonded to a carbon atom of the alicyclic or heteroalicyclic ring. Preferably, the ring consists of from four to eight atoms arranged in a closed ring such as, for example, a cycloalkane (i.e., a saturated ring structure) or a cycloalkene (i.e., a non-aromatic ring structure having at least one double bond). While alicyclic rings contain only carbon atoms as ring members, heteroalicyclic rings incorporate one or more heteroatoms in the ring structure. Heteroatoms for the present invention preferably include oxygen, nitrogen, and sulfur.

Novel compounds of the invention include both monocyclic compounds and polycyclic compounds. Monocyclic embodiments of the invention have one ring structure, namely an oxopentafluorosulfanyl-substituted alicyclic or heteroalicyclic ring. Polycyclic compounds have one or more additional rings directly or indirectly bonded to an oxopentafluorosulfanyl-substituted alicyclic or heteroalicyclic ring. The multiple rings of such polycyclic compounds are fused, bridged, or bridged-fused together, are spirocyclic, are linked via a single or double bond, or possess some combination of these.

Particularly preferred oxopentafluorosulfanyl-substituted alicyclic ring structures include cyclopropane, cyclopropene, cyclopentane, cyclopentene, cyclopentadiene, cyclohexane, cyclohexene, cyclohexadiene, cycloheptane, cycloheptene, cycloheptadiene, cycloheptatriene, cyclooctane, cyclooctene, cyclooctadiene, cyclooctatriene, and derivatives thereof. Particularly preferred oxopentafluorosulfanyl-substituted heteroalicyclic ring structures include thiophene, furan, pyran, pyrrole, imidazole, pyrazole, oxazole, thiazole, pyrrolidine, imidazolidine, quinuclidine, pyrazolidine, morpholine, and derivatives thereof.

As used herein, the term derivative means a compound or chemical structure having the same fundamental structure or underlying chemical basis as the relevant related compound. Such a derivative is not limited to a compound or chemical structure produced or obtained from the relevant related compound.

The oxopentafluorosulfanyl-substituted alicyclics and heteroalicyclics of the invention can also include additional substitutes. Although the type of substitute that can be practiced with the present invention is not particularly limited, preferred substituents include halogen; C₁ - C₆ substituted or unsubstituted, branched or straight alkyl; C₅ - C₇ substituted or unsubstituted aryl or heteroaryl; C₃ - C₈ aliphatic cyclic; carboxy; and oxopentafluorosulfanyl, with halogen being more preferred and fluorine, chlorine and/or bromine being even more preferred.

In certain preferred embodiments, the invention provides novel compounds having a structure according to Formula I: 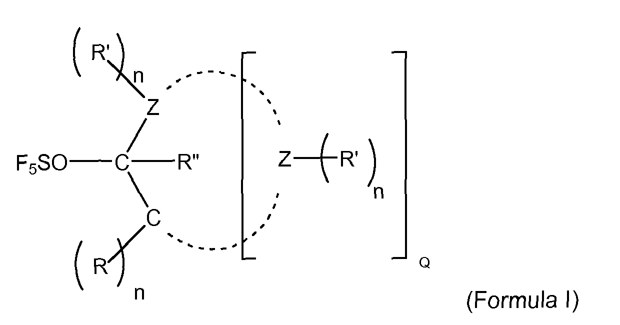 wherein
Z is independently selected from C, O, S, or N;
R, R', and R" are independently selected from H; halogen; C₁ - C₆ substituted or unsubstituted, branched or straight alkyl; C₅ - C₇ substituted or unsubstituted aryl or heteroaryl; C₃ - C₈ aliphatic cyclic; atom of a fused, bridged, fused-bridged, or spirocyclic ring structure; O; and OSF₅; provided that if R' is O or OSF₅, the corresponding Z is C and further provided that at least one of R, R', and R" is not fluorine;
Q is a positive integer from 1 to 5;
n is independently 0, 1, or 2; and
---- is independently a single or double covalent bond.

In certain highly preferred embodiments, compounds of the invention have a structure according to Formula II: 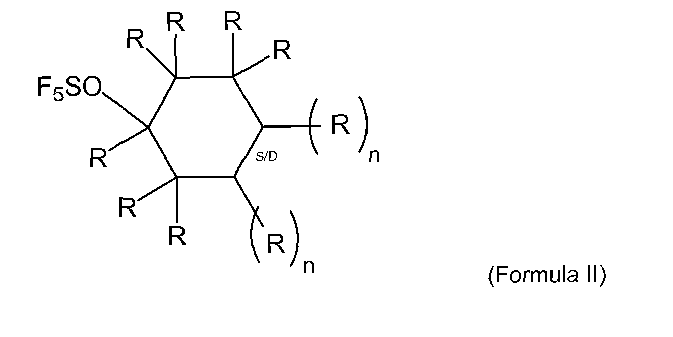 wherein
R is independently selected from H, F, Cl, Br, I, and OSF₅, with H, F, Cl, and Br being preferred, provided that at least one R is not fluorine;
n is independently 1 or 2; and
S/D is a single or double bond.

Compound having oxopentafluorosulfanyl-substituted alicyclic or heteroalicyclic rings can be prepared, for example, by reacting pentafluorosulfur hypohalite (SF₅-OX', wherein X' is F or CI) with a compound having a substituted or unsubstituted cycloalkene or heterocycloalkene ring structure.

Without being bound by a particular theory, it is believed that the oxopentafluorosulfanyl-substitution is achieved by heterolysis of the hypohalite's O-X' bond and electrophilic addition across a carbon-carbon double bond of the cycloalkene or heterocycloalkene ring structure. In particular, the X' halogen bonds to one of the carbon atoms and the SF₅O- group bonds to the other carbon.

Generally, reactions between cycloalkenes and hypofluorites are considered to be unstable. For example, many reactions involving these compounds typically lead to self-accelerating decomposition (i.e., detonation). However, the inventors have found that, according to the method of the present invention, cycloalkenes undergo a stable reaction with pentafluorosulfur hypohalites, including pentafluorosulfur hypofluorite, to form a surprisingly stable oxopentafluorosulfanyl-substituted product. These stable reactions include those involving non-perfluorinated cycloalkenes, which are generally more reactive than perfluorinated cycloalkenes, aryls, and alkenes. These results are surprising in view of the fact that the addition of pentafluorosulfur hypofluorite to even relatively stable compounds, such as tetrafluoroethylene, is difficult. (See, e.g., S. Williamson, et al., reporting that "An excessive rate of addition [of SF₅OF to C₂F₄] caused explosions.")

Suitable pentafluorosulfur hypohalites for the present invention include those having the structure of Formula III:

SF₅-OX' (Formula III)

wherein X' is F or Cl.

Particularly preferred is pentafluorosulfur hypochlorite.

In general, any substituted or unsubstituted cycloalkene or heterocycloalkene ring structure is suitable for the invention, and thus is not particularly limited. Preferred cycloalkene and heterocycloalkene ring structures have from 4 to 8 ring members. Suitable heteroatoms include oxygen, nitrogen, and sulfur.

A compound comprising the substituted or unsubstituted cycloalkene or heterocycloalkene ring structure can be monocyclic (i.e., having only one ring structure, namely the substituted or unsubstituted cycloalkene or heterocycloalkene) or polycyclic. Such polycyclic compounds have one or more additional rings directly or indirectly bonded to the substituted or unsubstituted cycloalkene or heterocycloalkene ring.

Examples of preferred monocylic compounds having a cycloalkene ring structure include cyclopropene, cyclopentene, cyclopentadiene, cyclohexene, cyclohexadiene, cycloheptene, cycloheptadiene, cycloheptatriene, cyclooctene, cyclooctadiene, and cyclooctatriene. More preferred are cyclopentene, cyclopentadiene, cyclohexene, and cyclohexadiene, with cyclohexene and cyclohexadiene being particularly preferred.

Examples of suitable polycyclic compounds having a cycloalkene ring structure include bicyclo[4.4.0]deca-2-ene, bicyclo[3.3.1]nona-2-ene, bicyclo[2.2.0]hexa-2,5-diene, tricyclo[3.1.0.0^{2,6}]hex-3-ene, spiro[3.5]nona-1,5,7-triene, bicyclohexadiene, and benzylcyclohexadiene.

The cycloalkene or heterocycloalkene ring structures can also have one or more substituted groups in addition to the oxopentafluorosulfanyl. Preferred substituents for substituted cycloalkene or heterocycloalkene include, but are not limited to, halogen, C₁ - C₆ substituted or unsubstituted, branched or straight alkyl, C₅ - C₇ substituted or unsubstituted aryl or heteroaryl, C₃ - C₈ aliphatic cyclic, and carboxyl. The cycloalkene or heterocycloalkene ring structures can also have two or more oxopentafluorosulfanyl groups.

In preferred embodiments, the compound having a cycloalkene or heterocycloalkene ring has a structure according to Formula IV: 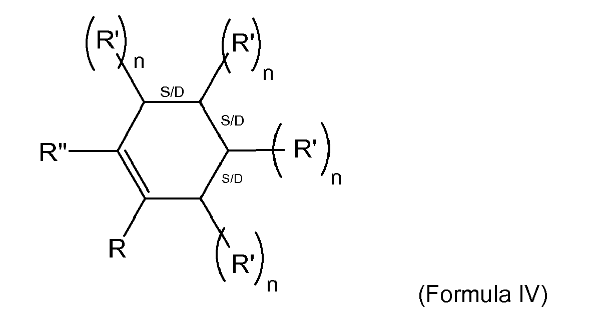 wherein
R, R', and R" are independently selected from H, halogen, C₁ - C₆ substituted or unsubstituted, branched or straight alkyl, C₅ - C₇ substituted or unsubstituted aryl or heteroaryl, C₃ - C₈ aliphatic cyclic, O, and OSF₅, provided that at least one of R, R', and R" is not F;
n is independently 1 or 2; and
S/D is independently a single or double bond, provided that the compound contains no more than two double bonds.

In particular preferred embodiments, R, R', and R" are independently H, F, Br, or Cl, and the ring contains one or two double bonds.

The combination of reaction time, temperature, and/or pressure required to achieve electrophilic addition of an oxopentafluorosulfanyl group to a compound having a cycloalkene or heterocycloalkene ring is dependent upon several factors, including for example the particular reactants involved and the desired yield. Such combinations of times, temperatures, and pressures can be determined by those skilled in the art without undue experimentation in view of the teachings contained herein. In certain preferred embodiments, such as those involving the addition of oxopentafluorosulfanyl to a substituted or unsubstituted cycloalkene or heterocycloalkene, preferred reaction conditions comprise providing a non-aqueous solution of cycloalkene or heterocycloalkene at a temperature of from -90° C to 0° C, more preferably from -70° C to -40° C, and introducing, preferably by vapor transferring, pentafluorosulfur hypochlorite into the non-aqueous solution to form a reaction mixture. The temperature of the reaction mixture is then preferably increased, preferably by allowing the mixture to warm by the heat of reaction and/or by application of external heat transfer, to a temperature of 0° C to 30° C, more preferably from 20° C to 25° C, even more preferably about ambient temperature, while the electrophilic addition reaction proceeds. Preferably, the reaction conditions are chosen to achieve a near quantitative yield of oxopentafluorosulfanyl-substituted alicyclic or heteroalicyclic compound.

In general, oxopentafluorosulfanyl-substitution can occur at any carbon-carbon double bond of the cycloalkene or heterocycloalkene ring. Thus, if the ring structure contains multiple carbon-carbon double bonds, more than one oxopentafluorosulfanyl-substitutions can be achieved.

The oxopentafluorosulfanyl-substituted alicyclic or heteroalicyclic compound(s) produced by this reaction can be further modified, for example by selective dehalogenation, to produce other novel compounds containing at least one oxopentafluorosulfanyl functional group.

### EXAMPLES

Certain aspects of the present invention are further illustrated, but are not limited by, the following examples.

### Example 1:

This example demonstrates a method for preparing 2,4,5-trichloro-1 - oxopentafluorosulfanylcyclohexane.

Approximately 3.312 g (21.9 mmol) of 4,5-dichlorocyclohex-1-ene and 30 mL anhydrous CHCl₃ was loaded into a 3-neck round-bottom flask under a N₂ atmosphere. The mixture was cooled to less than about -40 °C (using acetonitrile/CO₂) and stirred. Approximately 3.9 g (21.9 mmol) pentafluorosulfur hypochlorite (SF₅OCl), was vapor transferred into the stirred mixture to produce a reaction mixture. The reaction mixture was then allowed to warm to about ambient temperature and was then stirred under a N₂ atmosphere for approximately 60 minutes. After the 60 minutes elapsed, the mixture was heated to about 45 °C under vacuum to remove volatile components. As the remaining composition was cooled to about ambient temperature, a deposit of a white solid weighing 6.027 g formed. NMR analysis and GC-MS analysis of the solid confirmed that the product was 2,4,5-trichloro-1 -oxopentafluorosulfanylcyclohexane. The isolated yield was 84%.

### Example 2:

This example demonstrates a method for preparing 2-chloro-4,5-dibromo-1-oxopentafluorosulfanylcyclohexane.

Approximately 2.564 g (10.7 mmol) of 4,5-dibromocyclohex-1-ene and 50 mL anhydrous CHCl₃ was loaded into a 3-neck round-bottom flask under a N₂ atmosphere. The mixture was cooled to less than about -40 °C (using acetonitrile/CO₂) and stirred. Approximately 1.9 g (10.6 mmol) pentafluorosulfur hypochlorite (SF₅OCl), was vapor transferred into the stirred mixture to produce a reaction mixture. The reaction mixture was then allowed to warm to about ambient temperature and was then stirred under a N₂ atmosphere for approximately 30 minutes. After the 30 minutes elapsed, the mixture was heated to about 45 °C under vacuum to remove volatile components. As the remaining composition was cooled to about ambient temperature, a deposit of a white solid weighing 3.861 g formed. NMR analysis and GC-MS analysis of the solid confirmed that the product was 2-chloro-4,5-dibromo-1-oxopentafluorosulfanylcyclohexane. The isolated yield was 86%.

### Example 3:

This example demonstrates method for preparing 2-chloro-4,5-dibromo-1-oxopentafluorosulfanyl-cyclohexane on a larger scale than demonstrated in Example 2.

Approximately 18.129 g (76 mmol) of 4,5-dibromocyclohex-l-ene and 90 mL anhydrous CHCl₃ was loaded into a 3-neck round-bottom flask under a N₂ atmosphere. The mixture was cooled to less than about -40 °C (using acetonitrile/CO₂) and stirred. Approximately 13.6 g (76.2 mmol) pentafluorosulfur hypochlorite (SF₅OCl), was vapor transferred into the stirred mixture to produce a reaction mixture. The reaction mixture was then allowed to warm to about ambient temperature and was then stirred under a N₂ atmosphere for approximately 90 minutes. After the 90 minutes elapsed, the mixture was heated to about 45 °C under vacuum to remove volatile components. As the remaining composition was cooled to about ambient temperature, a deposit of a white solid weighing 26.619 g formed. NMR analysis and GC-MS analysis of the solid confirmed that the product was 2-chloro-4,5-dibromo-1-oxopentafluorosulfanylcyclohexane. The isolated yield was 84%.

### Example 4:

This example demonstrates a method for preparing 2-chloro-1-oxopentafluorosulfanyl-cyclohex-4-ene.

A solution of 2-chloro-4,5-dibromo-1-oxopentafluorosulfanylcyclohexane (15.36g, 36.75 mmol) in EtOH/THF (75mL/75mL) was loaded into a 3-neck 250 mL round-bottom flask equipped with a N₂ inlet tube, rubber septum and glass stopper. This solution was treated with zinc powder (9.70g, 147 mmol) and stirred under N₂ at ambient temperature for 16 hours to form an admixture. The admixture was filtered through celite and the permeate was diluted with EtOAc (100 mL), washed with water (3X25mL), dried using MgSO₄, filtered and evaporated *in-vacuo* to obtain 8.91 g (94% yield) of preparing 2-chloro-1-oxopentafluorosulfanyl-cyclohex-4-ene.

### Example 5:

This example demonstrates another method for preparing 2-chloro-1-oxopentafluorosulfanyl-cyclohex-4-ene. This method involves the direct addition of SF₅OCl to cyclohexadiene.

Approximately 4.169 g (52 mmol) of cyclohexadiene and 50 mL anhydrous CHCl₃ was loaded into a 3-neck round-bottom flask under a N₂ atmosphere. The mixture was cooled to less than about -40 °C (using acetonitrile/CO₂) and stirred. Approximately 9.1 g (51 mmol) pentafluorosulfur hypochlorite (SF₅OCl), was vapor transferred into the stirred mixture to produce a reaction mixture. The reaction mixture was then allowed to warm to about ambient temperature and was then stirred under a N₂ atmosphere for approximately 30 minutes. After the 30 minutes elapsed, the mixture was heated to about 25 °C under vacuum to remove volatile components. The remaining composition was cooled to about ambient temperature to form a colorless solid/liquid mixture weighing 10.698 g. NMR analysis and GC-MS analysis of the solid confirmed that the product was 2-chloro-1-oxopentafluorosulfanyl-cyclohex-4-ene.

### Example 6:

This example demonstrates a method for preparing 1-oxopentafluorosulfanyl-cyclohex-3-ene.

Approximately 258 mg (1 mmol) of 2-chloro-1-oxopentafluorosulfanyl-cyclohex-4-ene was charged into a 25 mL round-bottom flask equipped with a N₂ inlet tube, rubber septum and glass stopper. Approximately 582 mg (2 mmol) of (Bu)₃SnH and 2.5 mg (0.015mmol) of 2,2'-azobis(2-methylpropionitrile) (AIBN) were added and the mixture was heated to about 80°C for about 30 min under a nitrogen atmosphere. The mixture was then cooled to about ambient temperature and then distilled at 0.1 mm Hg to obtain 213 mg (95% yield) of 1-oxopentafluorosulfanyl-cyclohex-3-ene.

### Example 7:

This example demonstrates a method for preparing 3,4-dibromo-1-oxopentafluorosulfanyl-cyclohexane.

A solution of 1-oxopentafluorosulfanyl-cyclohex-3-ene (224 mg, 1 mmol) in CHCl₃ (3 mL) was loaded into a 3-neck 25 mL round-bottom flask equipped with a N₂ inlet tube, rubber septum and glass stopper. The solution cooled to about 0°C and then treated dropwise with bromine (160 mg, 1 mmol) under a nitrogen atmosphere. The mixture was stirred for about 10 min. After the 10 minutes had elapsed, the reaction was quenched by addition of saturated aqueous Na₂SO₃ (1 ml). The solution was diluted with CHCl₃ (10 mL) and the resulting aqueous phase was removed using a separatory funnel. The CHCl₃ solution was washed with brine (10 mL), dried (using MgS04), filtered and evaporated *in-vacuo* to obtain 371 mg (97% yield) of 3,4-dibromo-1-oxopentafluorosulfanyl-cyclohexane.

### Example 8:

This prophetic example will demonstrate a method for preparing 4,5-dichloro-2-fluoro-1-oxopentafluorosulfanylcyclohexane using pentafluorosulfur hypofluorite (SF₅OF) as a reactant.

The procedure described in Example 1 will be followed, except that SF₅OF is used as a reactant instead of SF₅OCl.

The results from this test will be comparable to the results obtained in Example 1.

Having thus described a few particular embodiments of the invention, it will be apparent to those skilled in the art, in view of the teachings contained herein, that various alterations, modifications, and improvements not specifically described are available. Accordingly, the foregoing description is by way of example only, and not limiting.

## Claims

1. A compound comprising at least one alicyclic or heteroalicyclic ring structure, the ring structure comprising a ring of four to eight atoms, wherein at least two adjacent atoms of the ring are carbon atoms and at least one of said carbon atoms is covalently bonded to an -OSF₅ functional group and either is bonded to an additional moiety selected from hydrogen; halogen; C₁ - C₆ substituted or unsubstituted, branched or straight alkyl; C₅ - C₇ substituted or unsubstituted aryl or heteroaryl; C₃ - C₈ aliphatic cyclic; OSF₅; or is a member of a fused, bridged, fused-bridged, or spirocyclic ring system; provided that if the additional moiety is fluorine, then at least one other ring atom is bonded to an atom or functional group other than fluorine.

2. The compound of claim 1, wherein said alicyclic or heterocyclic ring has a structure according to Formula I: 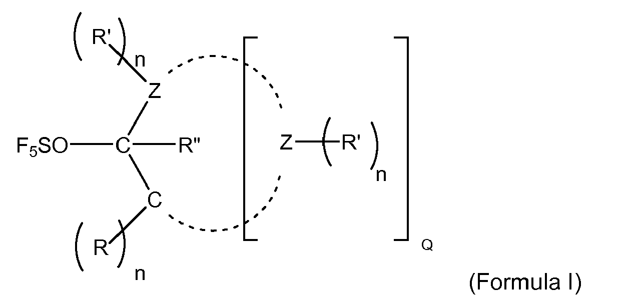 wherein
Z is independently selected from C, O, S, or N;
R, R', and R" are independently selected from H; halogen; C₁ - C₆ substituted or unsubstituted, branched or straight alkyl; C₅ - C₇ substituted or unsubstituted aryl or heteroaryl; C₃ - C₈ aliphatic cyclic; atom of a fused, bridged, fused-bridged, or spirocyclic ring structure; O; and OSF₅; provided that R" is not O, that if R' is O or OSF₅, the corresponding Z is C, and further that R, R', and R" are not all F;
Q is a positive integer from 1 to 5;
n is independently 0, 1, or 2; and
---- is independently a single or double covalent bond.

3. The compound of claim 2, wherein said alicyclic or heteroalicyclic ring has a structure according to Formula V: 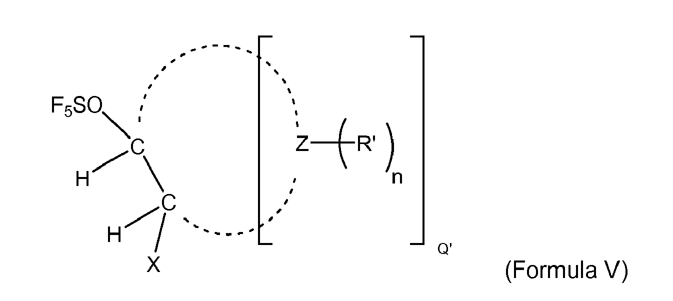 wherein
Z is independently selected from C, O, S, or N;
X is a halogen;
R' is independently selected from H; halogen; C₁ - C₆ substituted or unsubstituted, branched or straight alkyl; C₅ - C₇ substituted or unsubstituted aryl or heteroaryl; C₃ - C₈ aliphatic cyclic; atom of a fused, bridged, fused-bridged, or spirocyclic ring structure; O; and OSF₅; provided that if R' is O or OSF₅, the corresponding Z is C;
Q' is a positive integer from 2 to 6;
n is independently 1 or 2; and
---- is independently a single or double covalent bond.

4. The compound of claim 2 or 3 wherein at least one of said R' is halogen.

5. The compound of any one of claims 2 to 4 wherein R, R', and R" are independently selected from H and halogen.

6. The compound of any one of claims 2 to 5 wherein at least one pair of two adjacent Z's in said ring structure are covalently bonded via a double bond.

7. The compound of any preceding claim wherein said ring structure is a five- or six-member alicyclic or heteroalicyclic of a monocyclic system.

8. The compound of claim 7 having a structure according to Formula II: 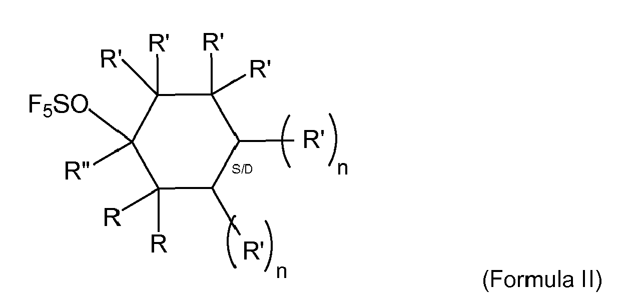 wherein
R, R', and R" are independently selected from H, F, Cl, Br, I, and OSF₅; provided that at least one R, R', or R" is not F;
n is 1 or 2; and
^{S/D} is a single or double bond.

9. The compound of claim 8 selected from:

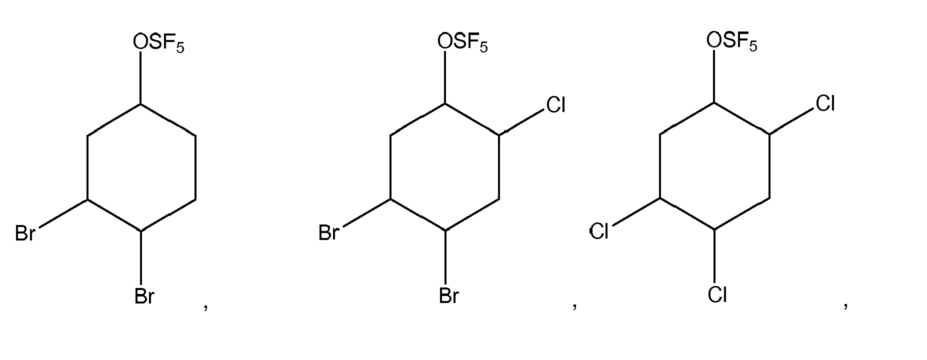

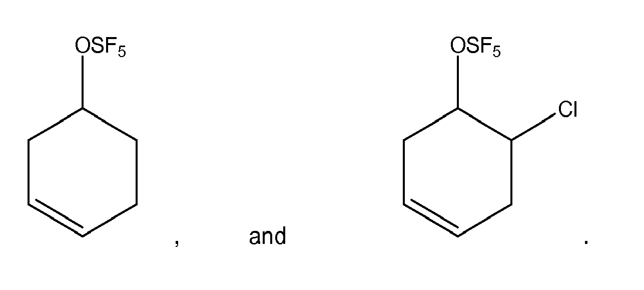

10. A method for preparing an oxopentafluorosulfanyl substituted compound comprising:
a. providing a reactant comprising a compound having a substituted or unsubstituted cycloalkene or heterocycloalkene ring, provided that said ring is not perfluorinated;
b. reacting said reactant with a pentafluorosulfur hypohalite to form a compound having an oxopentafluorosulfanyl-substituted alicyclic or heteroalicyclic ring.

11. The method of claim 10 wherein said reacting involves an electrophilic addition across a pair of double bonded carbons in said cycloalkene or heterocycloalkene ring.

12. The method of claim 10 or 11 wherein said pentafluorosulfur hypohalite has a formula of X'OSF₅, wherein X' is F or Cl.

13. The method of any one of claims 10 to 12 wherein said reactant comprises a compound having a four- to eight-member ring structure wherein said members are independently selected from C, N, O, and S.

14. The method of any one of claims 10 to 13 wherein said reactant comprises a substituted or unsubstituted cyclohexene or a substituted or unsubstituted cyclohexadiene.

15. The method of any one of claims 10 to 14, wherein the method is for preparing a compound according to any one of claims 1 to 9.

16. The method of claim 10 wherein said reactant comprises a cycloalkene having a structure according to Formula IV: 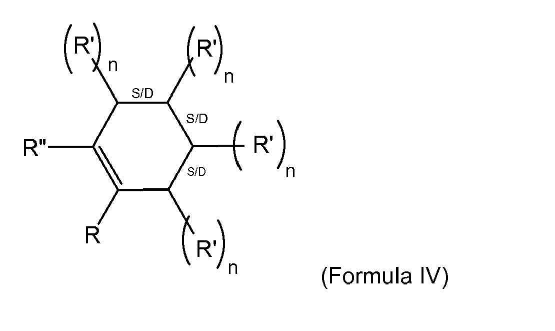 said pentafluorosulfur hypohalite is F₅SOCl, and
said alicyclic or heteroalicyclic ring has a structure according to Formula VI: 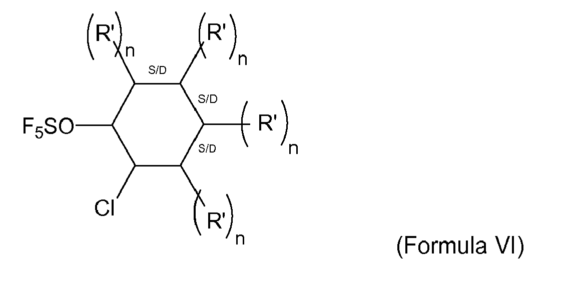 wherein
R and R" are hydrogen,
R' is independently selected from H, halogen, C₁ - C₆ substituted or unsubstituted, branched or straight alkyl, C₅ - C₇ substituted or unsubstituted aryl or heteroaryl, C₃ - C₈ aliphatic cyclic, and OSF₅;
n is independently 1 or 2; and
S/D is independently a single or double bond, provided that the ring does not contain an aromatic bond.

17. The method of claim 16, wherein said alicyclic or heteroalicyclic ring according to Formula VI is further subjected to one or more of:
a dehalogenating process wherein at least one halogen is removed from a member of the alicyclic or heteroalicyclic ring;
a dehalogenating process wherein a double covalent bond is formed between two adjacent members of the alicyclic or heteroalicyclic ring; and
a halogenation process wherein at least one halogen is added to one or more members of the alicyclic or heteroalicyclic ring.

18. A method for adding an oxopentafluorosulfanyl group to a compound comprising:
a. contacting a nonaqueous solution comprising a substituted or unsubstituted cycloalkene with a pentafluorosulfur hypochloride vapor at a temperature from -90° C to 0° C to form a reaction mixture; and
b. maintaining said reaction mixture at a temperature of from -90° C to 30° C to form an oxopentafluorosulfanyl-substituted alicyclic compound.
